# EUROPEAN PATENT APPLICATION

(11) **EP 4 434 508 A1**
(43) Date of publication of application: **25.09.2024**
(21) Application number: 23164003.8
(22) Date of filing: 24.03.2023
(51) Int. Cl.: A61H 9/00

(54) **SYSTEM FOR EXERCISING PRESSURE DRAINAGE AND COOLING OR WARMING OF ONE OR MORE PARTS OF THE BODY**

(71) Applicant: Di Stefano, Antonino, 95030 Pedara (CT) (IT)
(72) Inventor: DI STEFANO, ANTONINO, 95030 PEDARA (CT) (IT)
(74) Representative: Giuliano, Natalia

(57) **Abstract**

System (200) for exercising pressure drainage and cooling or warming of one or more parts of the body, comprising: a device (201) for performing compressive, peristaltic, sequential pressure drainage, modulated from 20 to 150 mmHg, under alternating thermal conditioning in "hot-cold/cold-hot" *sessions from -30 to +70 degrees centigrade, of one or more parts of the body in predetermined time intervals, simultaneously allowing peristaltic sequences on the limb/limbs to be treated; a pressure machine (202) connected to the device (201) for performing compressive, peristaltic, sequential pressure drainage and configurated to inject the compressed air into the device (201); and a cooling/warming machine (203) connected to the device for performing compressive, peristaltic, sequential pressure drainage and configurated to inject the compressed air into the device (201).

## Description

The present invention relates to a system for exercising pressure drainage and cooling or warming of one or more parts of the body.

In particular, the present invention relates to a system for exercising pressure drainage and cooling or warming of one or more parts of the body, for example: post-traumatic therapies, post-surgical therapies, vascular pathologies, aesthetic medicine, sports injuries, professional aesthetics.

As is known, the pressure drainage method, known by the term pressure therapy, is a treatment used in medicine or in aesthetics which is based on the exercise of pressure from the outside towards the inside on the patient's limbs, through a specific equipment. The areas that can be treated are normally the limbs and the abdominal area, using applicators, normally divided into several sections, at least four, which are worn by the patient, i.e. leggings, bracelets or abdominal band, and which are inflated sequentially from the outside towards the interior. Normally the single sectors of leggings, bracelets or abdominal bands have internal chambers (or bladders) made in interdependent sequential sections so as to allow a physiological emptying excluding any risk of reflux.

Known devices also allow the operating pressures of each individual sector to be adjusted both automatically and manually. The main fields of application of these devices are those of the treatment of venous or mixed lymphatic oedema, PEPS treatment, post-surgical oedema, post-traumatic oedema. The operation is simple and very effective: the pressure exerted by the inflating and deflating action of the air chambers of the leg, by means of a connected compressor, creates a deep sequential massage.

However, known pressotherapy machines do not allow to perform further functions on the treated limbs, other than that of applying pressure and cold to them.

This problem has been addressed in patent application WO2014151795, which relates to a rehabilitation system comprising an inner layer and an outer layer, comprising a first and a second portion, and arranged outside the inner layer. A therapeutic device, such as a cold wrap, configured to be placed in the inner layer is provided. The system further includes an adjustable closure mechanism connected to the first and second portions of the outer layer to adjust the compressive force exerted by the outer layer on a user's body.

However, this solution has the disadvantage that the cold layer must be placed in contact with the limb manually and that it must be removed again from the limb and refrigerated when it has lost its cooling effectiveness.

A second known solution is described in patent US5080089, which relates to an apparatus for the therapeutic treatment of injuries and bodily ailments, in particular by means of compression and by means of a fluid at non-ambient temperature, simultaneously or separately. The equipment is then operated in one of two modes. In one mode, non-ambient temperature fluid from a remote reservoir is continuously recirculated under pressure through a cuff wrapped around the wound. In another mode, a room temperature fluid is placed under pressure in the cuff and the cuff is sealed.

Another solution is described in patent application WO2013/190333 which reports a thermoregulation device for the treatment of injuries and physical ailments which includes a plurality of ducts equipped with flow constrictors which optimize the transfer of energy between the device and the patient. In particular, the described device comprises an outer shell configured to be wrapped around at least one part of a patient's body; at least one pressure membrane included and wrapped inside the outer shell and comprising air at a predetermined pressure; at least one thermal insulating layer positioned and wrapped inside the pressure membrane; and at least one cold membrane positioned and wrapped inside the thermal insulating layer and comprising at least one pipe in which a constantly refrigerated liquid flows.

Furthermore, EP1972312 discloses a device for increasing blood flow by applying pressure and/or controlling the applied temperature.

However, these solutions have the problem of not adequately distributing the liquid at a temperature other than room temperature uniformly. In fact, they do not have a way to convey the cold or hot alternatively and constantly, while maintaining pressure on the limb to be treated.

The object of the present invention is to provide a system for exercising pressure drainage and cooling or warming of one or more parts of the body which is capable of performing compressive, peristaltic, sequential, modulated under thermal conditioning alternating with hot-cold/cold-hot sessions of one or more parts of the body, namely pressure drainage and simultaneously conveying and maintaining the cold or hot alternatively in a constant manner, simultaneously allowing peristaltic sequences on the limb/limbs to be treated, in an effective and compact manner, therefore having characteristics such as to overcome the limits which still affect current systems with reference to the prior art.

According to the present invention, a system for exercising pressure drainage and cooling or warming of one or more parts of the body is provided, as defined in claim 1.

For a better understanding of the present invention, a preferred embodiment is now described, purely by way of non-limiting example, with reference to the attached drawings, in which:
- figure 1 shows a schematic view of a system for exercising pressure drainage and cooling or warming of one or more parts of the body, according to the invention;

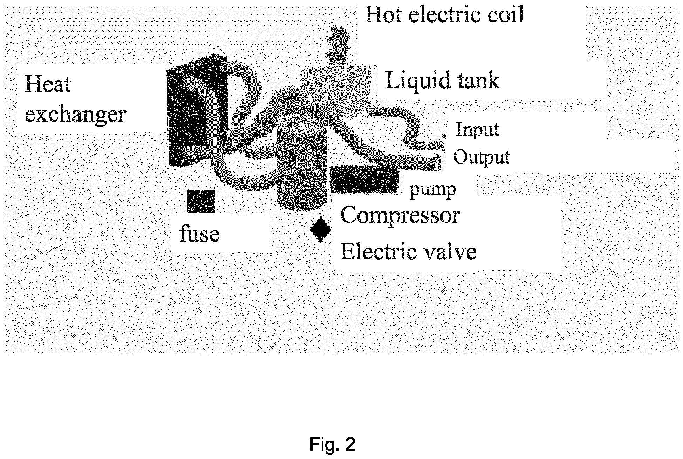

- figure 2 shows a schematic view of circuit to warm or cool one or more parts of the body integrated in the system for exercising pressure drainage and cooling or warming of one or more parts of the body, according to the invention;
- figure 3 shows a schematic view of a device for performing compressive, peristaltic, sequential pressure drainage comprised in the system for exercising pressure drainage and cooling or warming of one or more parts of the body, according to the invention;
- figure 4 shows a schematic view of the outer shell of device for performing compressive, peristaltic, sequential pressure drainage comprised in the system for exercising pressure drainage and cooling or warming of one or more parts of the body, according to the invention;
- figure 5 shows a schematic view of a pressure membrane comprised and wound inside the outer shell of the device for performing compressive, peristaltic, sequential pressure drainage, according to the invention;
- figure 6 shows a schematic view of a cold/warm membrane comprised and wound inside the outer shell of the device for performing compressive, peristaltic, sequential pressure drainage, according to the invention.

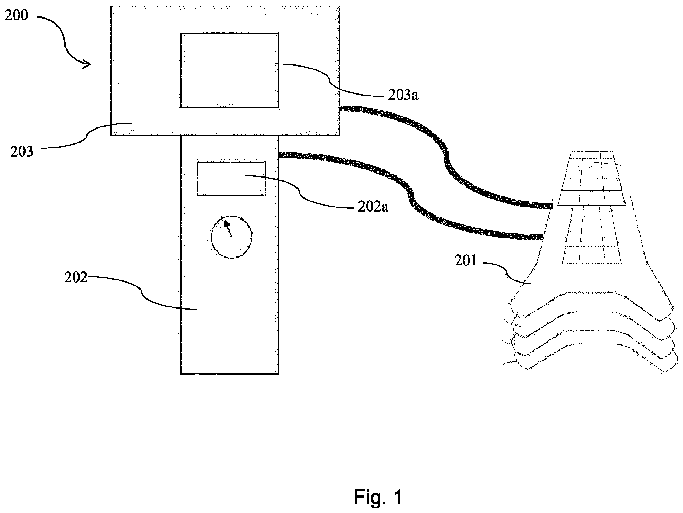

With reference to these figures and, in particular, to figure 1, a system for exercising pressure drainage and cooling or warming of one or more parts of the body is provided. In particular, the system 200 for exercising pressure drainage and cooling or warming of one or more parts of the body comprises:
- a device 201 for performing compressive, peristaltic, sequential pressure drainage, modulated from 20 to 150 mmHg, under alternating thermal conditioning in "hot-cold/cold-hot" sessions from -30 to +70 degrees centigrade, of one or more parts of the body in pre-determined time intervals, simultaneously allowing peristaltic sequences on the limb/limbs to be treated;
- a pressure machine 202 connected to the device 201 for performing compressive, peristaltic, sequential pressure drainage and configurated to inject the compressed air into the device 201; and
- a cooling/warming machine 203 connected to the device 201 for performing compressive, peristaltic, sequential pressure drainage configured to cool or warm the device 201.

According to an aspect of the invention, the device 201 for performing compressive, peristaltic, sequential pressure drainage is of the type described in the Italian patent N.102018000005404.

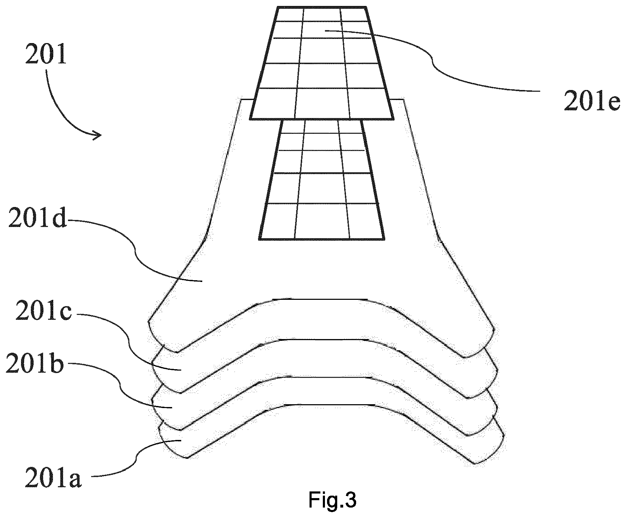

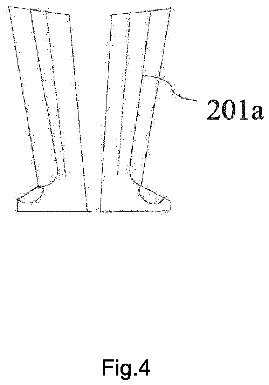

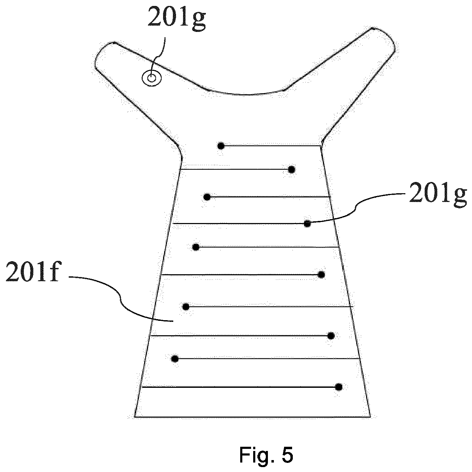

In particular, as showed in figures 3 and 4, the device 201 for exercising pressure drainage and warming/cooling of one or more parts of the body comprises an outer shell 201a, a pressure membrane 201b included and wrapped inside the outer shell 201a, a layer 201c of a thermal insulating material positioned and wound inside the pressure membrane 201b; - a cold/warm membrane 201d positioned and wrapped inside the thermal insulating layer 201c; and - cushioning means 201e positioned inside the cold membrane 201d and around the affected part of the body. More specifically, the pressure membrane 201b included inside the outer shell 201a, as showed in figure 5, is composed of a plurality of partially overlapping transversal chambers 201f each equipped with at least one valve 201g through which the pressure membrane 201b receives compressed air coming from the pressure machine 202 configured to send air at a pre-set pressure.

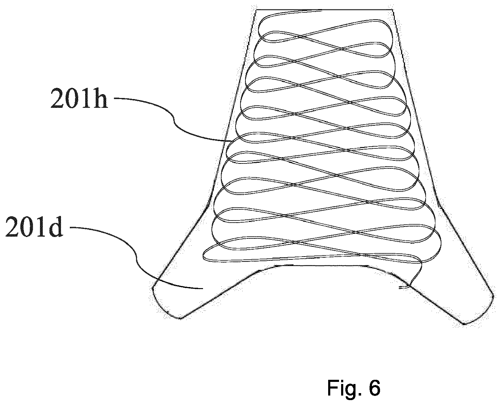

The thermal insulating material layer 201c has the role to keep cold or warm toward the parts of the body, without dispersing it toward the outer shell 201a. In fact, as showed in figure 6, the cold/warm membrane 201d, configured to generate and maintain a temperature of 70°C to -30°C, includes a coil tube 201h connected between an inlet valve and an outlet valve, not shown in the figure placed at the two opposite ends of the membrane 201d.

In more detail, according to one aspect of the invention, the cold membrane 201d internally comprises a plurality of sections in the longitudinal or transverse direction, within which portions of the serpentine tube 201g are included. This coil pipe 201h is connected by means of inlet and outlet valves to an inlet pipe of a liquid tank of the cooling/warming machine 203 containing a liquid, like propylene glycol, configured to be refrigerated and maintained at the desired temperature. The liquid can be, alternatively, warmed by a hot electric coil inserted in the tank and connected to an electric power source.

According to an aspect of the invention, the liquid tank and a compressor of the pressure machine 202 are connected to a heat exchanger.

According to one aspect of the invention, the coil 201h extends longitudinally along the cold membrane 201d; instead, according to another aspect of the invention, the coil 201h extends transversally along the cold membrane 201d.

According to one aspect of the invention, as shown in figure 1, the cushioning means 201e are a plurality of gel pads, configured to protect the limb or body part to be treated from possible traumas due to the pressure exerted by the serpentine tube 201h.

In the case, the system 200 ha to warm the device 201, an electric power source will warm the serpentine inside the liquid tank.

According to an aspect of the invention, the pressure machine 202 and the cooling/warming machine 203 are integrated into a single package.

According to an aspect of the invention, the cooling/warming machine 203 has a touch display 203a from which the user can insert the temperature parameters to cool or warm the device 201.

According to an aspect of the invention, the pressure machine 202 has a further touch display 202a from which the user can insert the temperature parameters to cool or warm the device 201.

According to an aspect of the invention, the pressure machine 202 comprises: a group of 10 or 20 solenoid valves for filling and emptying compressed air; a 5 bar and 80 liters per minute compressor; two silencers/filters; 12-pin pressure connector; emergency stop button; pressure reading with electronic transducer; 7-inch color touch screen compressed air unit.

According to an aspect of the invention, the cooling/warming machine 203 comprises: refrigeration unit 2-inch color touch screen; refrigerant unit pressure gauge; cooling group and heat exchanger; 5 lt griddle tank; 5L/min electric pump; 2500 W electrical resistance; 3400 W compressor; Pressure switch; solenoid valve; manual system for filling and emptying the grills; leggings shell; bracelet shell; gel shock absorbers; storage compartment.

In use, the device 201 is wrapped around the part of the body to be treated, for problems of a pathological, well-being or aesthetic nature. In particular, the part of the body to be treated is wrapped by the shell 201a. The part of the body to be treated will therefore receive the cold or hot due to the heat exchange produced by the cooling/warming machine 203 and the programmed peristaltic pressure sequences sent by the pressure machine 202. The cold/warm membrane 201d is connected, by means of piping suitably made of black closed-cell polyethylene, in such a way as to avoid the creation of condensation, to a machine for refrigerating a liquid, for example propylene glycol, sent by the aforementioned machine inside the coil tube 201h. In this way, cold is created around the entire body part to be treated. The cold is maintained, as the system is closed circuit. Furthermore, the dispersion of cold is prevented thanks to the layer of heat insulating material 201c wrapped around the cold/warm membrane 201d. Simultaneously with the creation of cold around the part to be treated, this is also subjected to the pressure generated by the pressure membrane 201b as it is connected, by its coil piping, to a compressor that generates compressed air. The outer shell 201a, which is a legging or a band of any other type to be wrapped around a limb of the patient's body, is wrapped around the pressure membrane 201b thus exerting a function of protection and adequate wrapping of the part to be treated.

Therefore, the system for performing compressive, peristaltic, sequential, modulated pressure drainage, under alternating thermal conditioning with "hot-cold/cold-hot" sessions, allows you to carry out a pressure drainage session by constantly modulating the part to be treated, as well as the pressures, even hot or cold temperature.

Furthermore, the system by system for exercising compressive, peristaltic, sequential, modulated pressure drainage, under alternating thermal conditioning with "hot-cold/cold-hot" sessions, avoids wasting water.

Furthermore, it can also be carried out on non-ambulatory and/or bedridden subjects, therefore without the need to go to rehabilitation centers.

Another advantage of the system for exercising compressive, peristaltic, sequential, modulated pressure drainage under alternating thermal conditioning with "hot-cold/cold-hot" sessions, is that it is simple and effective.

Another advantage of the system for exercising compressive, peristaltic, sequential, modulated pressure drainage, under alternating thermal conditioning with "hot-cold/cold-hot" sessions, of one or more parts of the body is its use in new fields such as vascular medicine, dermatological medicine, orthopedic medicine, aesthetic medicine and sports medicine.

Finally, the system for exercising compressive, peristaltic, sequential, modulated pressure drainage, under alternating thermal conditioning with "hot-cold/cold-hot" sessions, of one or more parts of the body is cheap.

Therefore, the system for exercising pressure drainage and cooling of one or more parts of the body according to the invention allows to carry out a pressure drainage session while constantly keeping the part to be treated, as well as under pressure, at a cold temperature.

Furthermore, the system for exercising pressure drainage and cooling of one or more parts of the body according to the invention avoids trauma to the part to be treated.

Furthermore, the system for exercising pressure drainage and cooling of one or more parts of the body according to the invention avoids the waste of water for filling tubs or swimming pools used in the current rehabilitation treatment known as the "Kneipp path".

Moreover, the system for exercising pressure drainage and cooling of one or more parts of the body according to the invention can also be carried out at home on non-ambulatory and/or bedridden subjects, therefore without the need to go to rehabilitation centers.

Another advantage of the system for exercising pressure drainage and cooling of one or more parts of the body according to the invention is that it is simple and effective.

Another advantage of the system for exercising pressure drainage and cooling of one or more parts of the body according to the invention is its use in new fields such as aesthetic medicine and sports medicine.

Finally, the system for exercising pressure drainage and cooling of one or more parts of the body according to the invention is economical.

Finally, it is clear that modifications and variations can be made to the system for exercising pressure drainage and cooling of one or more parts of the body described and illustrated here without departing from the protective scope of the present invention, as defined in the attached claims.

## Claims

1. System (200) for exercising pressure drainage and cooling or warming of one or more parts of the body, comprising:
- a device (201) for performing compressive, peristaltic, sequential pressure drainage, modulated from 20 to 150 mmHg, under alternating thermal conditioning in "hot-cold/cold-hot" *sessions from -30 to +70 degrees centigrade, of one or more parts of the body in pre-determined time intervals, simultaneously allowing peristaltic sequences on the limb/limbs to be treated;
- a pressure machine (202) connected to the device (201) for performing compressive, peristaltic, sequential pressure drainage and configurated to inject the compressed air into the device (201); and
- a cooling/warming machine (203) connected to the device for performing compressive, peristaltic, sequential pressure drainage and configurated to inject the compressed air into the device (201).

2. System (200) for exercising pressure drainage and cooling or warming of one or more parts of the body according to claim 1, **characterized in that** the device (201) for exercising pressure drainage and warming/cooling of one or more parts of the body comprises an outer shell (201a), a pressure membrane (201b) included and wrapped inside the outer shell (201a), a layer (201c) of a heat insulating material positioned and wound inside the pressure membrane (201b); - a cold/warm membrane (201d) positioned and wrapped inside the thermal insulating layer (201c); and - cushioning means (201e) positioned inside the cold membrane (201d) and around the affected part of the body. More specifically, the pressure membrane (201b) included inside the outer shell (201a) is composed of a plurality of partially overlapping transversal chambers (201f) each equipped with at least one valve (201g) through which the pressure membrane (201b) receives compressed air coming from the pressure machine (202) configured to send air at a pre-set pressure.

3. System (200) for exercising pressure drainage and cooling or warming of one or more parts of the body according to claims 1 and 2, **characterized in that** the thermal insulating material layer (201c) is configured to keep cold or warm toward the parts of the body, without dispersing it toward the outer shell (201a), the cold or warm being generated into the cold/warm membrane 201d by the cooling/warming machine (203), configured to generate and maintain a temperature of 70°C to -30°C, into a coil tube (201h) of the cold/warm membrane connected between an inlet valve and an outlet valve placed at the two opposite ends of the membrane (201d), the coil pipe (201h) being connected by means of inlet and outlet valves to an inlet pipe of a liquid tank of the cooling/warming machine (203) containing a liquid configured to be refrigerated and maintained at the desired temperature and to be, alternatively, warmed by a hot electric coil inserted in the tank and connected to an electric power source.

4. System (200) for exercising pressure drainage and cooling or warming of one or more parts of the body according to previous claims, **characterized in that** the liquid tank and a compressor of the pressure machine (202) are connected to a heat exchanger.

5. System (200) for exercising pressure drainage and cooling or warming of one or more parts of the body according to claim 1, **characterized in that** the pressure machine (202) and the cooling/warming machine (203) are integrated into a single package.

6. System (200) for exercising pressure drainage and cooling or warming of one or more parts of the body according to claim 1, **characterized in that** the cooling/warming machine (203) has a touch display (203a) from which the user can insert the temperature parameters to cool or warm the device (201).

7. System (200) for exercising pressure drainage and cooling or warming of one or more parts of the body according to claim 1, **characterized in that** the pressure machine (202) has a further touch display (202a) from which the user can insert the temperature parameters to cool or warm the device (201).

8. System (200) for exercising pressure drainage and cooling or warming of one or more parts of the body according to claim 1, **characterized in that** the pressure machine (202) comprises: a group of 10 or 20 solenoid valves for filling and emptying compressed air; a 5 bar and 80 liters per minute compressor; two silencers/filters; 12-pin pressure connector; emergency stop button; pressure reading with electronic transducer; 7-inch color touch screen compressed air unit.

9. System (200) for exercising pressure drainage and cooling or warming of one or more parts of the body according to claim 1, **characterized in that** the cooling/warming machine (203) comprises: refrigeration unit 2-inch color touch screen; refrigerant unit pressure gauge; cooling group and heat exchanger; 5 liter griddle tank; 5L/min electric pump; 2500 W electrical resistance; 3400 W compressor; Pressure switch; solenoid valve; manual system for filling and emptying the grills; leggings shell; bracelet shell; gel shock absorbers; storage compartment.
